# EUROPEAN PATENT APPLICATION

(11) **EP 2 082 682 A2**
(43) Date of publication of application: **29.07.2009**
(21) Application number: 09001002.6
(22) Date of filing: 26.01.2009
(51) Int. Cl.: A61B 1/31, A61B 17/34, A61B 19/00

(54) **Operating anoscope for transanal endoscopic microsurgery**

(30) Priority: 24.01.2008 US 62162 P
(71) Applicant: Karl Storz GmbH & Co. KG, 78532 Tuttlingen (DE)
(72) Inventor: Wexner, Steven David, Parkland, FL 33076 (US); Regadas, Sergio Frasisco Pinheiro, 60810-180 Fortaleza- Ceara (BR)
(74) Representative: Weller, Wolfgang

(57) **Abstract**

A surgical kit and method for transanal endoscopic surgical techniques. A medical instrument having an obturator tip with an insufflation channel and a tube for an optical device. A medical instrument having an insufflation channel and tube for an optical device, whereby a pressure system is used, such that auxiliary tools can be used to remove a tumor from a transanal cavity. A method for using the surgical kit and medical instruments individually and/or in sequence, whereby said surgical kit and medical instruments are used to access the transanal cavity by using a natural opening of the body.

## Description

The present application claims the benefit under 35 U.S.C. §119 (e) of the Provisional Patent Application Serial No. 61/062,162 filed January 24, 2008.

The present invention relates an apparatus and method for transanal endoscopic surgical techniques. More particularly, the present invention involves a medical instrument having an optical element for viewing, an obturator and insufflation channel for expanding, and a pressure system for maintaining the pressure in a transanal cavity, allowing a surgeon to insert and use auxiliary tools through the medical instrument to remove a tumor from the cavity. The invention further relates to a method for using the apparatus.

The technique of transanal endoscopic microsurgery (TEM) has been made available for clinical use since 1983. This technique is currently the only one-port system in endoscopic surgery by which there is a direct endoluminal approach to the target organ by using a natural opening of the body. The technique is useful in removing cancerous cells located in the rectal area or anus, which cause rectal cancer, and more specifically colon cancer or cancer of the intestines.

TEM involves a surgeon using a rectoscope, also known as a proctoscope, and having the surgeon operate by accessing the transanal cavity of a person. The surgeon is thus able to access the affected region through the use of the rectoscope. A rectoscope a short (10in or 25 cm long), straight, rigid, hollow metal tube, and typically has a small light bulb mounted at the end.

During TEM, a surgeon uses tools configured for the rectoscope to access the affected region. This allows the surgeon to access the affected region without having to make incisions into the body, specifically the transanal cavity or colon, to access the affected cancerous area. This, thus, provides greater ease, and comfort for the patient, while also being a less expensive and less intrusive procedure than one involving surgical incisions to access the affected area. Furthermore, a surgical procedure involving surgical incisions is more demanding upon the body of a patient to recover from, as well as being a higher risk surgery for the patient, as incisions can increase the risk of infection and have other side effects. Thus, transanal endoscopic microsurgery (TEM) is a valuable surgical technique with a low complication rate for patients. In particular, TEM is an efficient method for patients with adenomatous rectal tumors and early rectal cancer.

With TEM surgery, auxiliary tools are introduced through the rectoscope cylinder and into transanal cavity. These auxiliary tools are used by doctors to remove and treat the affected areas. However, problems exist whereby it can be difficult for surgeons to use auxiliary tools in the transanal cavity. One problem is that surgeons have limited vision, as it is difficult to see into the transanal cavity when working on the affected area. Other such problems involve inserting the rectoscope into the transanal cavity, and maintaining a pressure seal so it is easy to work in the cavity, while keeping the transanal cavity in an expanded state.

In the prior art, rectoscopes were designed to include optical devices, allowing for a surgeon to closely see the affected cancerous area. In particular, United States Patent No. 6,458,077, Boebel et al., teaches a rectoscope that performs this function, as Boebel teaches a channel that serves to introduce an optical element. However, Boebel suffers from the second problem that surgeons have in regard to rectoscopes, which involves insertion of the rectoscope into the transanal cavity. Particularly, Boebel does not disclose an obturator tip that allows for easy insertion of the rectoscope.

To get a rectoscope comfortably into the transanal cavity, surgeons typically use an obturator. An obturator, which is the central removable core of a rectoscope, allows for the easy insertion of the tip into the anus or another orifice.

During proctoscopy, the rectoscope is lubricated and inserted into the rectum. The obturator typically has a rounded end which protrudes through the far opening of the instrument. When inserted into the transanal cavity, the obturator expands the transanal cavity, thus allowing the surgeon to more easily access the cavity.

In the prior art, if a surgeon wanted to view the transanal cavity, the obturator would have be withdrawn, so that once the obturator was removed, the surgeon would have an unobstructed view of the interior of the transanal cavity.

Thus, the prior art teaches a method and apparatus whereby a surgeon would have a series of steps: first the surgeon would use an obturator for easy insertion of the rectoscope into the transanal cavity; second, the surgeon would have to remove the obturator; and third, the surgeon would have to insert an optical element into the rectoscope for viewing the interior of the affected transanal cavity. This series of steps is problematic and difficult for the surgeon to undertake.

Prior art designs attempted to solve this problem by developing a rectoscope that has removable parts. This allows a surgeon to mount an instrument with an obturator to the rectoscope. Once the transanal cavity was expanded, the surgeon would remove the obturator instrument and replace it with an instrument without an obturator, thus allowing for unobstructed access to the transanal cavity.

Using these methods, however, the surgeon would still have to first expand the transanal cavity using an obturator, remove the obturator, which is removable, while keeping the rectoscope lodged in the transanal cavity. The surgeon would then insert an optical device to find the affected area. This optical device would be removably linked to the rectoscope.

What is therefore desired is an invention that allows a surgeon to both expand the transanal cavity using the obturator and also to simultaneously view the affected area of the transanal cavity using an optical device. This makes it easier to find the affected area and to position the rectoscope accordingly, so that when a second optical device is inserted without the obturator and with auxiliary tools, the rectoscope is positioned well in relation to the affected area.

Furthermore, prior art such as Boebel discloses an insufflation channel in order to expand a body cavity. Insufflation involves an inert, nontoxic gas, such as carbon dioxide, being introduced into a body cavity, to expand the cavity. Insufflation is a common method to introduce compressed air into the transanal cavity, thus expanding the transanal cavity and reducing obstruction during investigative surgery.

While prior art, such as Boebel, discloses an insufflation channel, the prior art fails to disclose an insufflation channel in use with an obturator, or an obturator with a passage for insufflation. Rather, the prior art would require a surgeon to choose to apply either an insufflation channel or an obturator but not an instrument that has both elements.

What is therefore desired is an invention that allows a surgeon to both insufflate a transanal cavity, while simultaneously using an obturator to expand the transanal cavity.

When a transanal cavity is insufflate, pressure builds up in the cavity. It is important to maintain the increased pressure in the cavity in order to maintain the transanal cavity in the expanded state. Problems would result if carbon dioxide or other inert gases, which were provided during the insufflation process, would leak out through the transanal cavity. In particular, Boebel introduces a sealing mechanism to prevent the pressurized gas from being displaced from the transanal cavity.

However, Boebel, as well as other designs in the prior art, have limitations as problems result when auxiliary tools are used. In particular, when auxiliary tools are used, pressure can be released, which may cause the expanded transanal cavity to decrease in size.

The design of Boebel teaches a sealing element with a carrier element, but does not teach a sealing element that is fused into the mounting piece. Boebel further does not disclose a gasket and valve type fused assembly whereby the auxiliary tools continue through gaskets and valves in order to form a sealing chamber.

What is desired therefore is to have a gasket and valve pressure system whereby the auxiliary tools can be used easily and without losing the sealing pressure in the transanal cavity as well as in the housing of the rectoscope. It is further desired to have an obturator used with an optical device as well as having an obturator used simultaneously with an insufflation channel.

Accordingly, it is an object of the present invention to provide a medical instrument and method that allows a surgeon to both expand the transanal cavity using the obturator and also to simultaneously view the affected area of the transanal cavity using an optical device. It is a further object of the invention to provide a medical instrument and method that allows a surgeon to both insufflate a transanal cavity, while simultaneously using an obturator to expand the transanal cavity. It is a further object of this invention to provide an apparatus and method for a pressure system assembly having gaskets and valves, whereby auxiliary tools can be used easily and without losing the sealing pressure in the transanal cavity as well as in the housing of a rectoscope.

These and other objectives are achieved by providing a medical instrument comprising an insufflation channel comprising an insufflation channel, a tube for insertion of an optical device, and an obturator tip, said obturator tip having a first passage for receiving at least a potion of said insufflation channel and a second passage for receiving at least a portion of said tube.

The medical instrument may further allow for insufflation to occur simultaneously with insertion of the obturator tip into a transanal cavity. Furthermore, the tube in the medical instrument requires no special light source for the optical device.

In preferred embodiments, the insufflation channel and tube are fixed into the obturator tip. This may occur at the proximal or distal end of the insufflation channel and tube.

The medical instrument further may comprise a housing, which is typically in the shape of a cylinder. The medical instrument further may comprise a boss element, the boss element able to attach to the housing. The boss element is cap shaped in a preferred embodiment. Further, the housing may substantially house the insufflation channel and the tube, whereby the insufflation channel and tube are housed inside the housing cylinder.

In a referred embodiment, the obturator tip is bullet-shaped.

The medical instrument may also include a handle. Uses for the medical instrument include, but are not limited to, insufflation and expansion of a transanal cavity.

In a second embodiment, the present invention provides a medical instrument comprising an insufflation channel comprising a housing, a tube for insertion of an optical device, and a boss element, the boss element receiving the insufflation channel and tube, and the boss element attaching to the housing to seal the pressure within the housing.

The medical instrument may further comprise a pressure system allowing for insertion of auxiliary tools through the boss element and into the housing, while maintaining the pressure in the housing.

In a preferred embodiment, the pressure system has at least one gasket and at least one valve used in series. In other embodiments, the pressure system has one or more gaskets and in other embodiments the pressure system has one or more valves. It is preferable that the gaskets and valves work together to form a pressure chamber allowing for the insertion of auxiliary tools.

The boss element of the medical instrument may be cap-shaped. The tube in the medical instrument is typically straight and the auxiliary tools are curved.

In a third embodiment of the present invention, the present invention provides a surgical kit comprising a first medical instrument having an insufflation channel, a tube for insertion of an optical device, and an obturator tip, the obturator tip having a first passage for receiving at least a portion of the insufflation channel and a second passage for receiving at least a portion of the tube; a second medical instrument having an insufflation channel, a tube for insertion of an optical device, and a boss element, the boss element receiving the insufflation channel and the tube; and a housing for receiving the first medical instrument and the second medical instrument, wherein only one of the first medical instrument and the second medical instrument can be received by the housing at a given time, and wherein the boss element of the second medical instrument seals the pressure within the housing.

The second medical instrument allows for insertion of auxiliary tools through the boss element of the second medical instrument into the housing, while maintaining the pressure in the housing.

In preferred embodiments, the housing is preferably in the shape of a cylinder, and preferably the distal end of the housing is angled relative to the longitudinal axis of the housing.

The housing may have a locking mechanism to lock the housing into the first medical instrument or into the second medical instrument. The housing can substantially house the first or second medical instrument.

In preferred embodiments, the auxiliary tools are curved and the tube in both the first and second medical instruments is straight.

The fourth embodiment of the present invention provides a method for introducing a medical instrument into a transanal cavity, the medical instrument comprising a rectoscope attached to an obturator, wherein the obturator is used for expansion of the transanal cavity, and wherein the obturator has a first passage for insufflation and a second passage for an optical device.

The method further comprises the step of removing the obturator from the medical instrument, while maintaining the rectoscope in the transanal cavity.

The method further comprises the step of attaching a working element to the rectoscope, the working element having a sealing mechanism that allows sealing the pressure in the rectoscope. The sealing mechanism allows for inserting tools through the rectoscope and into the transanal cavity while maintaining the pressure in the rectoscope and the transanal cavity.

The sealing mechanism preferably has at least at least one gasket and at least one valve used in series. The sealing mechanism can involve more than one gasket and valve in order to conduct the sealing mechanism.

The fifth embodiment of the present invention provides a seal system comprising a surface having a passage with a proximal end and a distal end, the passage having a first member on the proximal end of the passage and a second member on the distal end of the passage; wherein the passage maintains a pressure drop between its proximal and distal ends, and wherein the passage is adapted to receive instruments.

The seal system has a first position and a second position, wherein in the first position the second member is closed to maintain the pressure drop in the passage, and wherein in the second position an instrument is inserted into the passage through the first member and the second member, opening the second closure member, whereupon the first member seals to maintain the pressure drop in said passage.

The seal system further may have a third position whereby the instrument is removed from the passage, and whereupon when the instrument is removed through the second member, but not the first member, the second member closes to maintain the pressure drop in the passage.

The seal system may involve the first member being a gasket and the second member being a valve.

Advantages for the above embodiments involve a new surgical kit with the ability to popularize transanal endoscopic excisions, thereby becoming easier, less expensive and more feasible in hospitals around the world.

Other objects of the invention and its particular features and advantages will become more apparent from consideration of the following drawings and accompanying detailed description. It should be understood that the detailed description and specific examples, while indicating the preferred embodiment of the invention, are intended for purposes of illustration only and are not intended to limit the scope of the invention.

FIG. 1 is a perspective view of the surgical kit including the housing and medical instruments of an embodiment of the present invention;

FIG. 2 is perspective view of the housing (rectoscope) of an embodiment of the present invention;

FIG. 3 is a right side view of the housing of FIG. 2 of the present invention;

FIG. 4 is a left side view of the housing of FIG. 2 of the present invention;

FIG. 5 is a perspective view of the medical instrument of an embodiment of the present invention;

FIG. 6 is a end view of the medical instrument of FIG. 5 of the preset invention;

FIG. 7 is a scaled close-up view of FIG. 5 of the present invention focused on the obturator tip;

FIG. 8 is a perspective view of the medical instrument of a second embodiment of the present invention;

FIG. 9 is a left side view of the medical instrument shown in FIG. 8 of the present invention focused on the spring valves;

FIG. 10 is a right side view of the medical instrument shown in FIG. 8 of the present invention focused on the gaskets;

FIG. 11 is a perspective view of an assembly of the present invention involving the housing (rectoscope) and medical instrument of FIG. 5 of an embodiment of the present invention;

FIG. 12 is a perspective view of an assembly of the present invention involving the housing (rectoscope) and medical instrument of FIG. 8 of an embodiment of the present invention;

FIG. 13 is a perspective view of the assembly with housing connected to the medical instrument of FIG. 8 of an embodiment of the present invention further showing the use of auxiliary tools;

FIG. 14 is a close-up view of the perspective view of FIG. 13 where the pressure system (gasket/valve) and linking mechanism of the housing to medical instrument of an embodiment of the present invention.

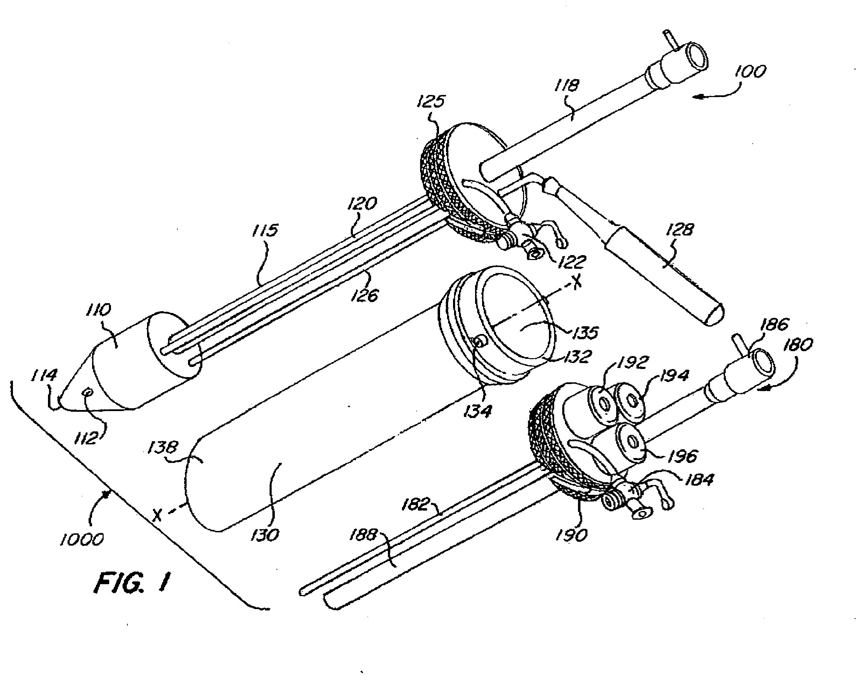

Referring to FIG. 1, a surgical kit assembly 1000 in accordance with an embodiment of the present invention is shown. This surgical kit assembly 1000 provides a method and apparatus for transanal endoscopic surgical techniques.

FIG. 1 shows medical instrument 100, rectoscope 130, and medical instrument 180. Medical instrument 100 includes obturator tip 110, insufflation channel 120, and tube 115. Tube 115 does not require a special light source that is usually required for optical devices used with rectoscopes. Boss element 125 is shown holding insufflation channel 120 and tube 115, forming the structure of medical instrument 100. In a preferred embodiment, insufflation channel 120 and tube 115 are shown parallel, or substantially parallel, to each other.

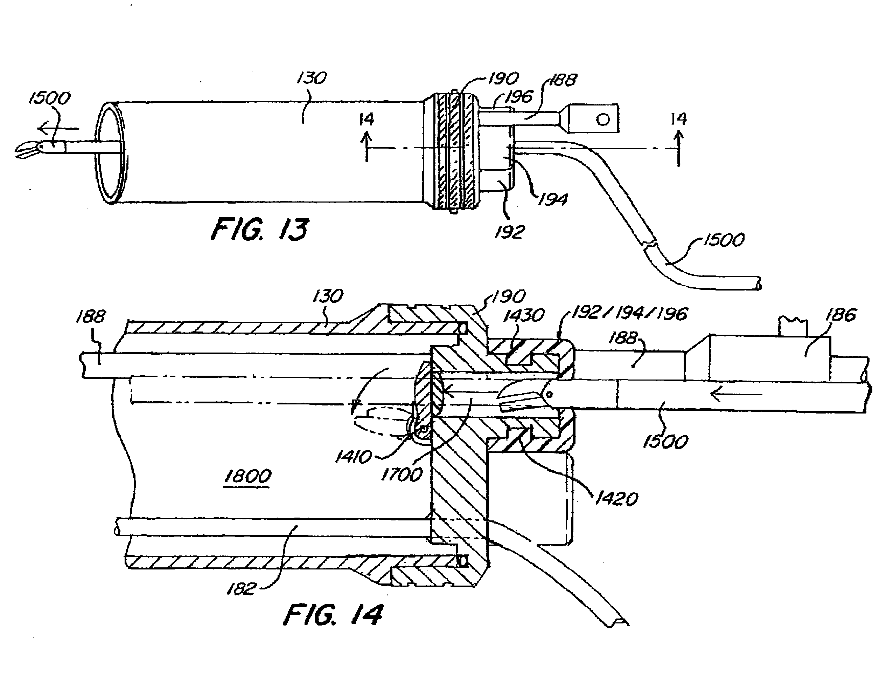

In a preferred embodiment, tube 115 is straight whereby auxiliary tools 1500 (shown in FIG. 13) are curved. The invention eliminates the need for a special angled telescope and instead any preexisting 5mm diameter, 10 mm diameter or other diameter telescope can be used.

FIG. 1 further shows obturator tip 110 containing first passage 112, which is shown inside the body of obturator tip 110. First passage 112 is shown corresponding to insufflation channel 120 which is shown running in an axial direction. Insufflation channel 120 has an end portion 122, which may be angled away from boss element 125, so that it is easier to introduce gases and insufflate transanal cavity 1100 (not shown). Insufflation channel 120 is shown going through boss element 125, which holds insufflation channel 120. In a preferred embodiment, insufflation channel 120 is sealed by boss element 125 so that pressure is not released from transanal cavity 1100 through the connection of the insufflation channel 120 and boss element 125. In another preferred embodiment, insufflation channel 120 is affixed to boss element 125.

Obturator tip 110 also contains second passage 114 corresponding to the distal end of tube 115, which is shown running in an axial direction parallel to insufflation channel 120. Second passage 114 is shown inside the body of obturator tip 110. Tube 115 is shown going through boss element 125. The end of tube 115 is shown to be element 118, which is the proximal side of tube 115. Tube 115 provides a means for insertion of an optical device 1200 (not shown). Optical device 1200 may be an endoscope, camera, or any such optical device used in the art. Optical device 1200 may be linked to a computer, such that a view of transanal cavity 1100 may be displayed on a surgical monitor.

As such, obturator tip 110 has first passage 112 for receiving at least a portion of insufflation channel 120 and second passage 114 for receiving at least a portion of tube 115.

In a preferred embodiment, surgical kit 1000 may be used with any type of compatible endoscope and laparoscopic CO₂ insufflator for transanal surgical procedures.

In another preferred embodiment, tube 115 may be sealed by boss element 125 such that pressure is not released from transanal cavity 1100 through the connection of tube 115 and boss element 125. In another preferred embodiment, tube 115 may be affixed to boss element 125.

Boss element 125 may be shaped as a cap. The cap allows housing 130 to be connected to boss element 125, allowing for housing 130 to substantially house insufflation channel 120 and tube 115.

Optionally, medical instrument 100 has stability column 126, shown connecting obturator tip 110 and boss element 125. Stability column 126 is shown connected to handle 128, although handle 128 can be connected to boss element 125, whereby no stability column 126 is needed. Neither stability column 126 nor handle 128 are essential within the scope and spirit of the invention, but can be used in preferred embodiments. Handle 128 may be used to remove medical instrument 100 from housing 130. Stability column 126 may provide structural stability to medical instrument 100 and maintain a mechanical connection between obturator tip 110 and boss element 125.

FIG. 1 also shows housing 130; typically referred to as a rectoscope. Housing 130 has a proximal end 132 and a distal end 138. Distal end 138 may be angled in a direction that is not perpendicular to longitudinal axis X of housing 130. The angle may be up to 80 degrees from perpendicular, or any angle in which housing 130 may be inserted into transanal cavity 1100, and may still function as a rectoscope. In a preferred embodiment, the angle is approximately 15 degrees from perpendicular; allowing for comfortable insertion of housing 130 into transanal cavity 1100 (not shown).

Proximal end 132 of housing 130 may include a linking means 134 whereby medical instrument 100 and medical instrument 180 may be secured to housing 130. In preferred embodiments, both medical instrument 100 and medical instrument 180 may lock into housing 130; however, this is not required.

Proximal end 132 of housing 130 may also gradually increase in diameter, although this is not a requirement. Furthermore, internal diameter 135 of housing 130 is shown, whereby internal diameter 135 is equal throughout the axial length of housing 130. However, in other embodiments of the invention, internal diameter 135 may vary along the axial length of housing 130.

The housing 130 typically has a smooth or polished surface so that during insertion, transanal cavity 1100 is not irritated or damaged. Housing 130 may be made of various materials including metal alloys, polymers, and other such materials known in the art.

FIG. 1 further displays medical instrument 180. Medical instrument 180 may include an insufflation channel 182 connected to boss element 190. Insufflation channel 182 is shown with end piece 184, allowing for insufflation of transanal cavity 1100 (not shown).

Medical instrument 180 may further include a tube 188 connected to boss element 190, whereby tube 188 has end piece 186 for inserting an optical device. The optical device could be an endoscope, camera, or any such optical device used in the art. The optical device may be linked to a computer, such that a view of the transanal cavity 1100 (not shown) may be shown on a surgical monitor.

Furthermore, end piece 186 of tube 184 is shown providing a surgeon with a view of transanal cavity 1100. A piece of glass or prism may be inserted into tube end piece 186 or tube 188, allowing a surgeon to look at a direction perpendicular or substantially perpendicular to tube 188 while still having an unobstructed view of transanal cavity 1100.

Boss element 190 may further include gaskets 192, 194, and 196. More or fewer gaskets may be used in keeping with the scope and spirit of the invention. In the preferred embodiment shown, three gaskets are used.

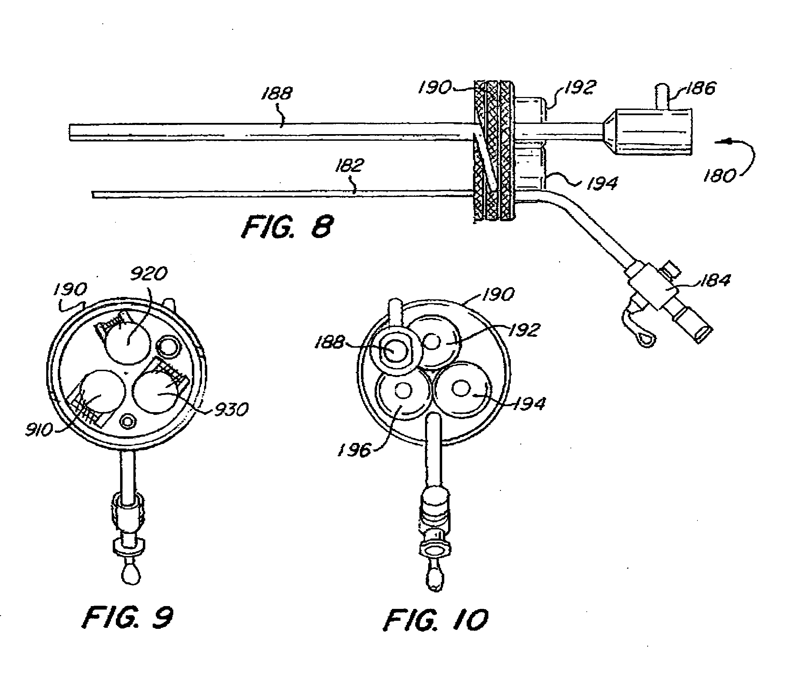

Boss element 190 further includes spring valves 910, 920, and 930 (shown in FIG. 9). These spring valves typically correspond to gaskets 192, 194, and 196, as shown in FIG. 1. More or fewer spring valves may be used in keeping with the scope and spirit of the invention. In the preferred embodiment shown, however, three spring valves 910, 920, and 930 are used.

The gaskets 192, 194, and 196 and spring valves 91.0, 920, and 930 allow for auxiliary tools 1500 (shown in FIGS. 13 and 14) to be used by a surgeon within the transanal cavity 1100. The gaskets and spring valves allow for the use of auxiliary tools 1500 without losing pressure within the insufflated transanal cavity 1100. The spring valves 910, 920, and 930 open to the inside of housing 130, and can be popped open as auxiliary tools 1500 are inserted through boss 190 and into the internal part of housing 130.

In a preferred embodiment, tube 188 is sealed by boss element 190 such that pressure is not released from transanal cavity 1100 through the connection of tube 188 and boss element 190. In another preferred embodiment, tube 188 is affixed to boss element 190. Similarly, in a preferred embodiment, insufflation channel 182 is sealed by boss element 190 and may be affixed to boss element 190.

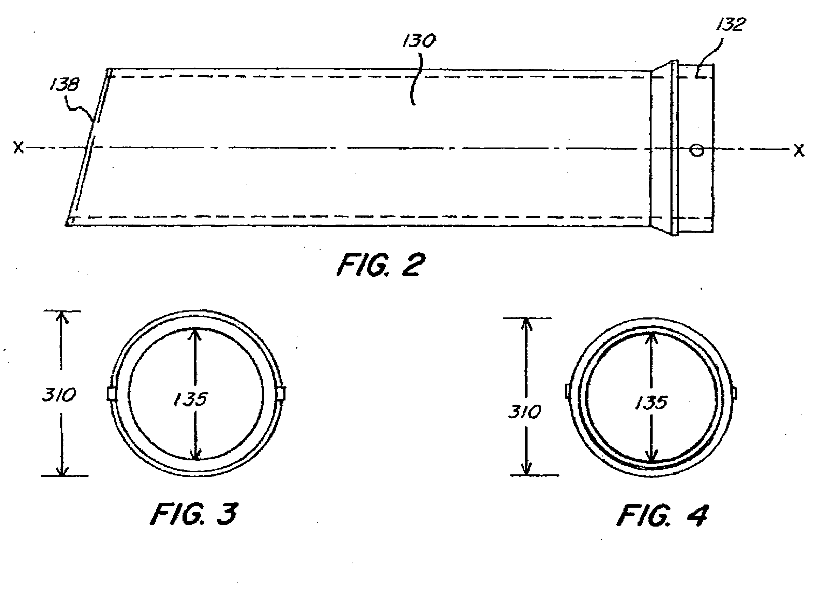

FIG. 2 shows a close up view of housing 130 whereby distal end 138 is shown angled and proximal end 132 is shown having a gradually increasing diameter. Distal end 138 is shown non-perpendicular to longitudinal axis X of housing 130. This allows for easier access to transanal cavity 1100.

FIG. 3 shows a left end view and FIG. 4 shows a right end view of housing 130, also known as the rectoscope. Internal diameter 135 is shown, whereby external diameter 310 is also shown being larger than internal diameter 135. Proximal end 132 contains linking means 134 which attaches housing 130 to medical instrument 100 and medical instrument 180. Different types of linking means 134 may be used including a screwing action, whereby a male part enters a female part. Other types of linking means 134 include suction, grooves which fit a male part into a female part, and any other types of coupling mechanisms known in the art that can removably connect housing 130 to medical instrument 100 and medical instrument 180.

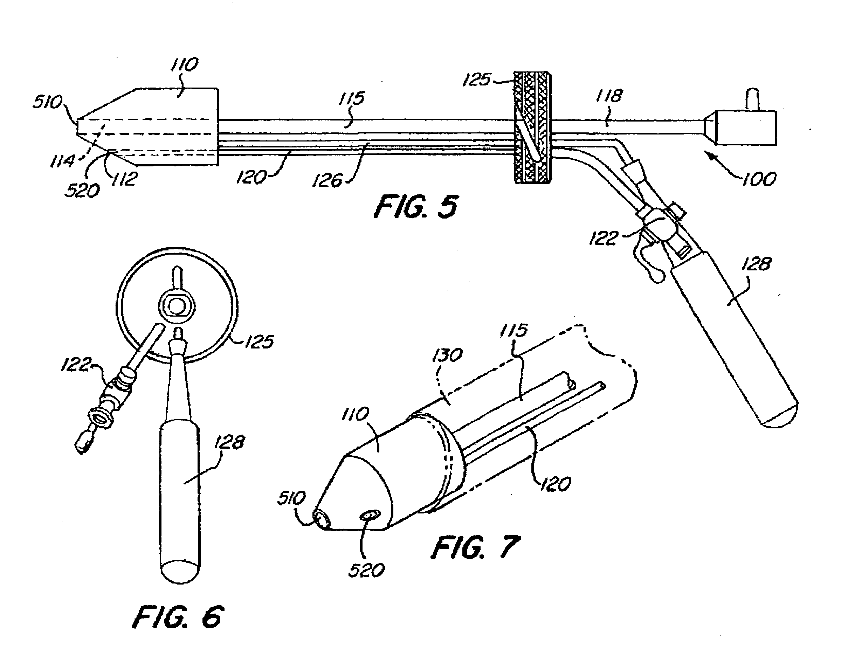

FIG. 5 shows a perspective view of medical instrument 100. In particular, FIG. 5 focuses on obturator tip 110. Obturator tip 110 may include a first passage 112, which is shown inside the body of obturator tip 110. Exit point 520 is shown whereby at this point, carbon dioxide or other inert gases are insufflated into transanal cavity 1100.

FIG. 5 also focuses on second passage 114 corresponding to the distal end of tube 115. Second passage 114 has exit point 510, whereby at this portion of obturator 110, an optical device can be used to see the affected area of transanal cavity 1100. This arrangement allows a surgeon to both insufflate a transanal cavity 1100 while using obturator 110 to expand the cavity as well, while simultaneously using tube 115 having an optical device to view the affected area.

In another embodiment of the invention, medical instrument 100 can have obturator tip 110 and insufflation channel 120, but not contain tube 115. In another embodiment of the invention, medical instrument 100 can have obturator tip 110 and tube 115, but not contain insufflation channel 120. However, the preferred embodiment includes the obturator tip 110, insufflation channel 120, and tube 115 as elements of medical instrument 100.

FIG. 6 shows an end view of medical instrument 100. Here end piece 122 of insufflation channel 120 is shown, whereby gases to insufflate transanal cavity 1100 may be introduced. Handle 128 is also shown, which may be used to insert and secure medical instrument 100 to the housing 130. Additionally, handle 128 may be used to introduce medical instrument 100, with housing 130 attached, into the transanal cavity 1100.

FIG. 7 displays a close-up view of obturator tip 110 enclosed in housing 130, whereby medical instrument 100 is shown substantially covered by housing 130. Here, insufflation channel 120 is shown corresponding to first passage 112 of obturator tip 110, and tube 115 is shown corresponding to second passage 114 of obturator tip 110. Obturator tip 110 is also shown whereby housing 130 has an angled distal end 138 supporting the weight of obturator tip 110.

FIG. 8 shows medical instrument 180. Here, the focus is on the pressure system 1600 (not shown in figures) that contains a combination of gaskets and spring valves.

Pressure system 1600, also known as a port valve system, allows for auxiliary tools 1500 to be used with a rectoscope 130 and maintains a pressure seal. The pressure is maintained in transanal cavity 1100 when a surgeon uses auxiliary tools 1500 to treat affected cancerous area in transanal cavity 1100.

Pressure system 1600 uses different types of gaskets and valves. The valves shown in FIG. 9 are spring valves 910, 920, and 930, but can also be other types of valves, such as solenoid valves, two-way and three-way valves, ball valves, hydraulic/pneumatic valves, and other valve systems understood in the art.

FIG. 9 shows a view of spring valves 910, 920, and 930 and FIG. 10 shows their corresponding gaskets 196, 192, and 194. In a preferred embodiment of the invention, gaskets 196, 192, 194 are shown corresponding to valves 910, 920, 930, although this is not require. Spring valves 910, 920, and 930 typically open to internal area 1800 of housing 130 (shown in FIG. 14).

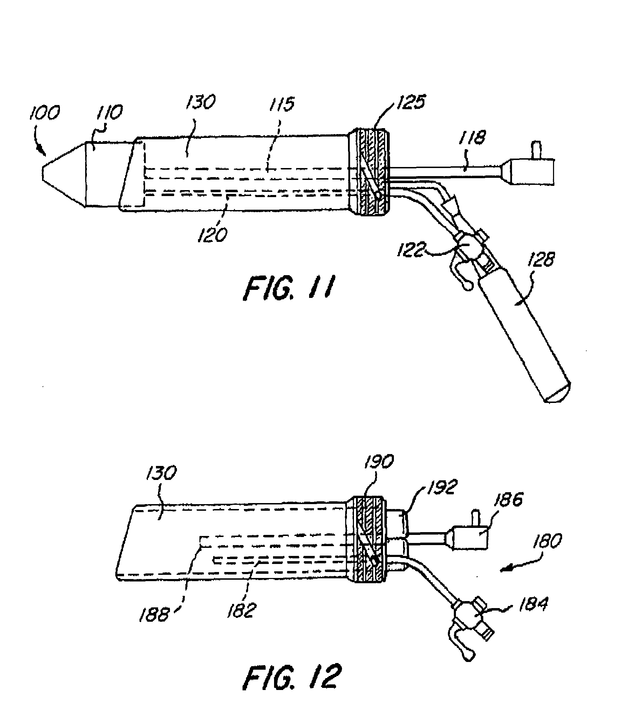

FIGS. 11-12 show an assembly of surgical kit 1000, whereby medical instrument 100 and medical instrument 180, are shown assembled within housing 130. Specifically, FIG. 11 shows surgical kit 1000 whereby insufflation channel 120 and tube 115 of medical instrument 100 are shown substantially enclosed by housing 130. FIG. 12 shows insufflation channel 182 and tube 188 of medical instrument 180, substantially enclosed by housing 130.

FIGS. 13-14 show medical instrument 180 assembled with the housing 130, whereby elements of pressure system 1600 are shown allowing use of auxiliary tools 1500.

In FIG. 13, auxiliary tool 1500 is shown going through gasket 194/196, and further through housing 130. This allows the end of auxiliary tool 1500 to reach transanal cavity 1100 and allows a surgeon to treat the affected section of transanal cavity 1100.

FIG. 14 shows a close-up cross-sectional view of the sealing mechanism of pressure system 1600 whereby a pressure chamber 1700 is formed. Here, auxiliary tool 1500 is shown entering gasket 192/194/196. Gasket 192/194/196 includes air lock elements 1420 and 1430, which provide a seal around auxiliary tool 1500 to prevent gas from seeping back out of internal area 1800 of housing 130, and/or transanal cavity 1100. As the auxiliary tool 1500 is inserted through the pressure chamber 1700, spring valve 1410 is opened via the pressure of the auxiliary tool 1500. After auxiliary tool 1500 passes through spring valve 1410, auxiliary tool 1500 passes into internal area 1800 of housing 130. Auxiliary tool 1500 can then be pushed through internal area 1800 of housing 130 until it reaches the distal end 138 of housing 130. Auxiliary tool 1500 then passes distal end 138 and is applied to the affected area of transanal cavity 1100. This allows the surgeon to access the affected tumor.

Pressure chamber 1700 is formed via the combination of gasket 192/194/196 and spring valve 1410. This creates the sealing means and prevents pressure from leaving housing 130, thus keeping transanal cavity 1100 insufflated and expanded, allowing for easier access for the surgeon to conduct the endoscopic surgery. Furthermore, the sealing means is a novelty of the present invention, as the pressure system 1600 involves an "air lock" using spring valve 1410 and gasket 192/194/196 in series. When auxiliary tools 1500 are not inserted into housing 130, spring valve 1410 remains in a closed position, thus sealing internal area 1800 of housing 130 from outside atmospheric pressure. This maintains the pressure difference in pressure chamber 1700 when no instruments are inserted into pressure chamber 1700.

When auxiliary tool 1500 is inserted into pressure chamber 1700, auxiliary tool 1500 first goes through gasket 192/194/196, which includes air lock elements 1420 and 1430. Auxiliary tool 1500 then goes through spring valve 1410, causing spring valve 1410 to open. At this point, air lock elements 1420 and 1430 function to provide a seal, thus providing an "air lock" and preventing pressure in internal area 1800 of housing 130 from escaping. This maintains the pressure difference in pressure chamber 1700 when an instrument 1500 is inserted into pressure chamber 1700.

When auxiliary tool 1500 is removed, it first is pulled back through spring valve 1410. As auxiliary tool 1500 is fully pulled through spring valve 1410, the spring valve 1410 closes. This prevents pressure from being released and maintains the pressure difference in pressure chamber 1700 when an instrument is through the gasket 192/194/196, but not the valve 1410.

Once spring valve 1410 has closed, air lock elements 1420 and 1430 are released, so that auxiliary tool 1500 can be pulled through gasket 192/194/196. Thus, pressure system 1600 remains air tight both when spring valve 1410 is in an open or closed position, and functions as an "air lock."

Thus, a surgeon can insert a first auxiliary tool 1500 into a gasket, remove first auxiliary tool 1500, and insert a second auxiliary tool into the same gasket without losing pressure in internal area 1800 of housing 130. A surgeon can thus use a plethora of different auxiliary tools 1500 in a surgery without having each gasket correspond to an individual auxiliary tool, providing increased flexibility during surgery.

FIG. 14 also shows insufflation channel 182 and tube 188 going through the internal area 1800 of housing 130. These elements lead to the affected transanal cavity 1100, as insufflation channel 182 provides for insufflation and tube 188 provides for viewing of the affected area of transanal cavity 1100. This allows the surgeon to keep transanal cavity 1100 expanded while being able to view the affected area via the optical device in tube 188.

In preferred embodiments, insufflation channel 120 is typically thinner than tube 115 for optical instruments. Insufflation channel 120 is also known as an irrigation channel.

Preferred embodiments also allow for auxiliary tools 1500 to be flexible. Also insufflation channels 120 and 182 and tubes 115 and 188 can be flexible as well, allowing for ease of use of endoscopic surgery.

Surgical kit 1000 and medical instruments 100 and 180, as well as housing 130 can be made of metals, alloys, plastics, polymers, and other such materials understood to be used for surgical devices.

In preferred embodiments, boss element 125 of medical instrument 100 and boss element 190 of medical instrument 180 have a larger diameter than housing 130, so that housing 130 can be inserted into medical instrument 100 and medical instrument 180, respectively. Another advantage to the present invention is that medical instrument 100 can be removed from housing 130 without losing insufflation pressure in transanal cavity 1100 and in internal area 1800 of housing 130.

The present invention further comprises a method using medical devices 100 and 180 in conjunction with housing 130. The method for transanal surgical procedure comprises the steps of introducing a medical instrument 3000 (no ref. no. 3000 in drawings) into transanal cavity 1100, medical instrument 3000 having a rectoscope 130 attached to obturator element 110, wherein obturator element 110 is used for expansion of transanal cavity 1100, and wherein obturator element 110 has a passage 112 for insufflation. The obturator also has a second passage 114 for viewing the affected area of transanal cavity 1100. An insufflation channel 120 and tube 115 correspond to the first passage 112 and second passage 114 of the obturator respectively. Tube 115 allows the surgeon to locate the affected area and position rectoscope 130 such that it is in the correct position for accessing the affected area.

The method further can comprise removing obturator element 110 from medical instrument 3000, while maintaining the position of rectoscope 130 in transanal cavity 1100. This involves quickly performing this step such that pressure is not lost from transanal cavity 1100.

The method further comprises inserting a working element 180 into transanal cavity 1100, whereby working element 180 attaches to rectoscope 130, wherein working element 180 has a sealing mechanism that allows for inserting auxiliary tools 1500 into transanal cavity 1100 while maintaining pressure in transanal cavity 1100 and housing 130.

The method further can comprise the sealing mechanism of working device 180 using gaskets and valves. At least one gasket or valve component is used, and preferably the method involves using three corresponding gaskets and valves. The method further can comprises using gaskets and valves in series, such that pressure does not leave transanal cavity 1100 when auxiliary tools 1500 are used to access a tumor.

Further advantages of the present invention include popularizing transanal endoscopic excisions, thereby becoming easier, less expensive and more feasible in hospitals around the would.

While the invention has been specifically described in connection with certain specific embodiments thereof, it is to be understood that this is by way of illustration and not of limitation and that various changes and modifications in form and details can be made thereto, and the scope of the appended claims should be construed as broadly as the prior art will permit.

The description of the invention is merely exemplary in nature, and thus, variations that do not depart from the gist of the invention are intended to be within the scope of the invention. Such variations are not to be regarded as a departure from the spirit and scope of the invention.

## Claims

1. A medical instrument comprising:
an insufflation channel;
a tube for insertion of an optical device; and
an obturator tip, said obturator tip having a first passage for receiving at least a portion of said insufflation channel and a second passage for receiving at least a portion of said tube.

2. The medical instrument of Claim 1, wherein said tube requires no artificial light source for said optical device.

3. The medical instrument of Claim 1, wherein said insufflation channel and said tube are fixed into said obturator tip.

4. The medical instrument of Claim 1, further comprising a housing.

5. The medical instrument of Claim 4, further comprising a boss element attached to said insufflation channel and said tube, said boss element able to attach to said housing.

6. The medical instrument of Claim 5, wherein said housing substantially houses said insufflation channel and said tube.

7. The medical instrument of anyone of Claims 1 to 6, wherein said obturator tip is bullet-shaped.

8. The medical instrument of Claim 5, wherein said boss element is cap-shaped.

9. The medical instrument of anyone of Claims 1 to 8, further comprising a handle.

10. A medical instrument comprising:
a housing;
an insufflation channel;
a tube for insertion of an optical device; and
a boss element, said boss element receiving said insufflation channel and said tube, said boss element attaching to said housing to seal the pressure within said housing.

11. The medical instrument of Claim 10, further comprising a pressure system, said pressure system allowing for insertion of auxiliary tools through said boss element into said housing, while maintaining the pressure in said housing.

12. The medical instrument of Claim 11, wherein said pressure system has at least one gasket and at least one valve used in series.

13. The medical instrument of Claim 11, wherein said pressure system has one or more gaskets.

14. The medical instrument of Claim 11, wherein said pressure system has one or more valves.

15. The medical instrument of anyone of Claims 10 to 14, wherein said boss element is cap-shaped.

16. The medical instrument of Claim 10, wherein said tube is straight.

17. The medical instrument of Claim 11, wherein said auxiliary tools are curved.

18. A surgical kit comprising:
a first medical instrument having an insufflation channel, a tube for insertion of an optical device, and an obturator tip, said obturator tip having a first passage for receiving at least a portion of said insufflation channel and a second passage for receiving at least a portion of said tube;
a second medical instrument having an insufflation channel, a tube for insertion of an optical device, and a boss element, said boss element receiving said insufflation channel and said tube;
a housing for receiving said first medical instrument and said second medical instrument, wherein only one of said first medical instrument and said second medical instrument can be received by said housing at a given time, and wherein said boss element of said second medical instrument seals the pressure within said housing.

19. The surgical kit of Claim 18, wherein said second medical instrument allows for insertion of auxiliary tools through said boss element of said second medical instrument into said housing, while maintaining the pressure in said housing.

20. The surgical kit of Claim 18, wherein said housing is shaped as a cylinder, and wherein the distal end of said housing is angled relative to the longitudinal axis of said housing.

21. The surgical kit of Claim 18, wherein said housing has a locking mechanism to lock said housing into said first medical instrument or into said second medical instrument.

22. The surgical kit of Claim 18, wherein said housing substantially houses said first medical instrument or said second medical instrument.

23. The surgical kit of Claim 18, wherein said auxiliary tools are curved.

24. The surgical kit of Claim 18, wherein said tube for insertion of an optical device of said first medical instrument and said second medical instrument is straight.

25. A seal system comprising:
a surface having a passage with a Proximal end and a distal end, said passage having a first member on the proximal end of said passage and a second member on the distal end of said passage;
wherein said passage maintains a pressure drop between its proximal and distal ends, and wherein said passage is adapted to receive instruments.

26. The seal system of Claim 25 having a first position and a second position,
wherein in said first position said second member is closed to maintain the pressure drop in said passage,
and wherein in said second position an instrument inserted into said passage through said first member and said second member, opening said second closure member, whereupon said first member seals to maintain the pressure drop in said passage.

27. The seal system of Claim 26, wherein in a third position said instrument is removed from said passage, and whereupon when said instrument is removed through said second member, but not said first member, said second member closes to maintain the pressure drop in said passage.

28. The seal system of Claim 25, wherein said first member is a gasket.

29. The seal system of Claim 25, wherein said second member is a valve.
